# EUROPEAN PATENT APPLICATION

(11) **EP 2 898 842 A1**
(43) Date of publication of application: **29.07.2015**
(21) Application number: 14152116.1
(22) Date of filing: 22.01.2014
(51) Int. Cl.: A61B 17/122, A61B 17/128, A61B 17/06, A61B 17/064, A61B 17/00

(54) **Delivery of a clamping implant via a puncture**

(71) Applicant: Norden Medical B.V., 9301 NZ Roden (NL)
(72) Inventor: Abbring, Drewes Pieter, 9756 CE Glimmen (NL); Smits, Johan, 9301 JK Roden (NL); Schulting,, Edwin Alexander, 9751 VD Haren (NL); Kalmann, Menno, 8075 PD Elspeet (NL); Kaptein, Wieger Gerard, 3751 DL Bunschoten-Spakenburg (NL)
(74) Representative: Jansen, Cornelis Marinus

(57) **Abstract**

A tool for delivering and applying a clamping implant (9; 109) via a puncture in skin tissue (3) and underlying tissue (2) of a patient, comprising a hollow needle (4; 104) bounding a lumen (5; 105) and forming a helix, a plunger (6; 106) of which a distal portion extends in longitudinal direction inside the lumen (5; 105), and a plunger operating member (7; 107) engaging a proximal portion of the plunger (6; 106) projecting from the needle (4; 104), for screwing the plunger (6; 106) further into or out of the needle (4; 104) by screwing rotation of the needle (4; 104) relative to the plunger (6; 106). A system including such a tool and a method for delivering and applying a clamping implant (9; 109) using such an instrument are also described.

## Description

### FIELD AND BACKGROUND OF THE INVENTION

The invention relates to a tool, a system and a method for delivering and applying a clamping implant via a puncture in skin tissue and underlying tissue. The proposed tool, system and method are particularly suitable for closing off varicose veins, in particular the Great Saphenous Vein and the Small Saphenous Vein by clamping off. However, the proposed device, system and method can also be used for other treatments involving applying a clamp in tissue in a position spaced under the skin, such as embolization or clamping of a biliary duct, an ureter, a Fallopian tube etc.

Conventionally, such treatments include surgical interventions to create space for movement of the clip and the clip application tool in the tissue surrounding the hollow structure to be closed off. It is particularly challenging to achieve this with little tissue damage when approaching a tubular hollow structure in a direction about perpendicular to the longitudinal axis of that tubular structure. An alternative is not to create such free space, but to thread some cross stitches of surgical wire through the tissue. However a disadvantage of such an approach is that it frequently causes tears in the tissue, so that the tissue is still damaged.

More specifically, known surgical treatments of more-severe varicose veins that do not respond to self-care, include vein stripping (removing a long vein through small incisions), Ambulatory Phlebectomy (removal of smaller varicose veins using a hook through a series of 2-3 mm skin punctures), endoscopic vein surgery (a thin video camera inserted in the leg visualizes varicose veins, that are then closed off and removed through small incisions) and vein ligation (tying a piece of thread around the vein to shut it off).

In European patent application 2 599 450 a tool, a clip and a method for closing off a varicose vein is disclosed in which the clip is introduced to a location where the vein is to be closed by clamping via a needle punctured through the skin and through tissue between the skin and the vein to be treated. A problem associated with this method and device is that a substantial change in shape of the clip is required when the clip exits the needle, that, after exiting the needle, the clip has to be advanced through tissue surrounding the vein to be clamped and that after the deformation upon exiting the needle and having been advanced through tissue adjacent to the location where the vein is to be clamped, it has to be ensured that the clip exerts the clamping action required to close off the vein.

### SUMMARY OF THE INVENTION

It is an object of the invention to provide a simple solution that allows delivering a clamping implant through a patient's skin and through tissue under the skin in a minimally invasive manner, to a location spaced from the skin, where the clamping implant exerts a clamping action to tissue engaged by the clamping implant.

According to the invention, this object is achieved by providing a tool according to claim 1. The invention can also be embodied in a system according to claim 12, which comprising such a tool and a clamping implant and in a method according to claim 15.

Because the needle has a longitudinal axis forming a helix, the needle can be punctured to the location where the clamping implant is to be delivered along a helix shaped puncture path. The plunger operating member engaging a proximal portion of the plunger projecting from the needle, for screwing the plunger further into or out of the needle by screwing rotation of the needle relative to the plunger and the coiled clamping implant, allows the clamping implant to be delivered from the needle at the delivery location by screwing back the needle, while restraining the plunger from being screwed back with the needle, so that the plunger is held essentially stationary relative to the tissue surrounding the helix shaped puncture path. The plunger effectively allows the clamping implant in a position at a distance underneath the skin surface to be restrained from being screwed back with the needle.

Deformation of the clamping implant can remain limited to deformation to a biased state inside the lumen of the needle and a gradual release from the biased state inside the lumen of the needle to a clamping state in which the clamping implant engages tissue clamped thereby, as the clamping implant exits the needle that is screwed out.

Other features of embodiments of the invention are set forth in the dependent claims.

Further objects, features, effects and details of the invention are described below with reference to embodiments shown in the drawings.

### BRIEF DESCRIPTION OF THE DRAWINGS

Figs. 1-5 are schematic representations of an example of a tool, a system and a method according to the invention in successive stages of operation;
Fig. 6 is a side view of a second example of a tool according to the invention;
Fig. 7 is a perspective exploded view of an example of a system according to the invention including a tool as shown in Fig. 6;
Fig. 8 is a side view in cross-section along a center line of a system as shown in Fig. 7 in an operating condition prior to delivery of a clamping implant; and
Fig. 9 is a side view according to Fig. 8 of a tool as shown in Figs. 6-8 in an operation condition after delivery of a clamping implant.

### DETAILED DESCRIPTION

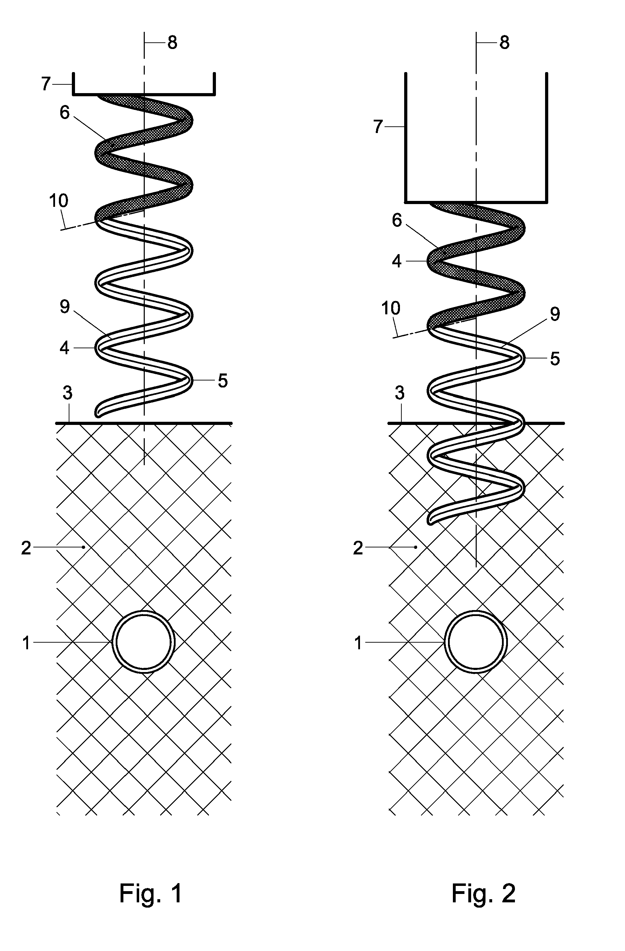

In Figs. 1-5 successive stages of operation of first examples of a tool, of a system, and of a method according to the invention are shown. In the present example, the application of the tool is for closing off a varicose vein 1 which is located in tissue 2 at a distance under skin tissue 3. The distance between the vein and the skin is typically 1 to 3 cm. The diameter of the vein 1 is usually 10 mm or less. A closing device for closing off the vein 1 will usually have to be passed through the skin 3 and through slightly less than 1 to 3 cm of underlying tissue 2.

Figs. 1-5 also show a first example of a tool and a system according to the invention in successive stage of operation. The tool is composed of a hollow needle 4 bounding a lumen 5 and having a longitudinal axis forming a helix, a plunger 6 (schematically represented as the fully black portion of the needle 4 proximally of to dash-and-dot line 10) of which a distal portion extends in longitudinal direction inside the lumen 5, and a plunger operating member 7. The plunger operating member 7 engages a proximal portion of the plunger 6 projecting proximally from the needle 4, for screwing the plunger 6 further into or out of the needle 4 by screwing rotation of the needle 4 relative to the plunger 6 about an axis (center line) 8 of the helix.

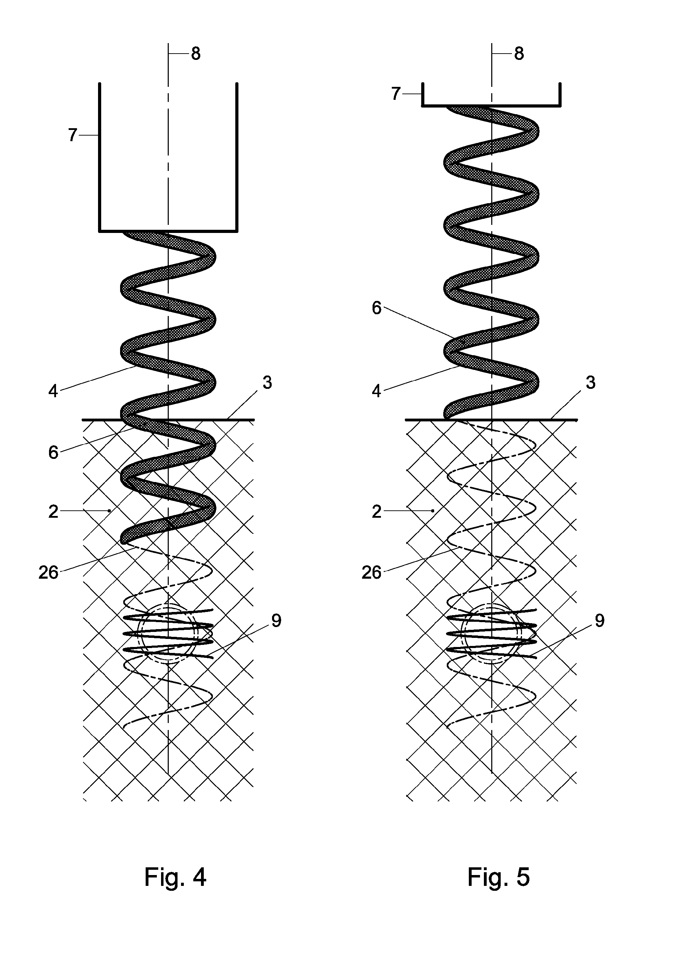

In addition to the tool, the system further includes a clamping implant 9 in the form of a coiled wire. In Figs.1-3, the clamping implant 9 in a biased configuration inside a distal end portion of the needle 4 is schematically represented by a line distally of the dash-and-dot line 10 and coaxial with the distal end portion of the needle 4. In Figs. 4 and 5, the clamping implant 9 clamping the tissue 2 under the skin 3 in an area spaced from the skin is schematically represented by a side view of the coiled wire. Because the tissue is resilient, it is compressed by the clamping force exerted by the clamping implant. This causes the biased clamping implant 9 to be released from the biased shape in the needle to a partially released configuration in in which the pitch between successive windings in axial direction (direction of the central axis 8) is substantially smaller than the pitch between successive windings in axial direction in the biased configuration.

The vein screw can be used to treat, for example, a varicose vein 1 in the Great Saphenous Vein and the Small Saphenous Vein. Prior to starting the procedure of delivering the clamping implant 9 to a location where the vein 1 is to be closed off, the location of the vein 1 is determined (e.g. using ultrasound) and for instance marked on the skin. The location may for instance be in the thigh near the inguinal area or in the popliteal fossa. The tissue 2 and the skin 3 into which the needle 4 is to be punctured are preferably locally anaesthetized. Then, the tool pre-loaded with a clamping implant 9 may be taken out of a packaging for keeping the tool sterile. Then, the system composed of the tool and the clamping implant 9 in a distal end portion of the needle 4 is positioned with a distal end of the needle at the determined location on the skin 3, preferably in an orientation about perpendicular to the skin, as illustrated by Fig. 1.

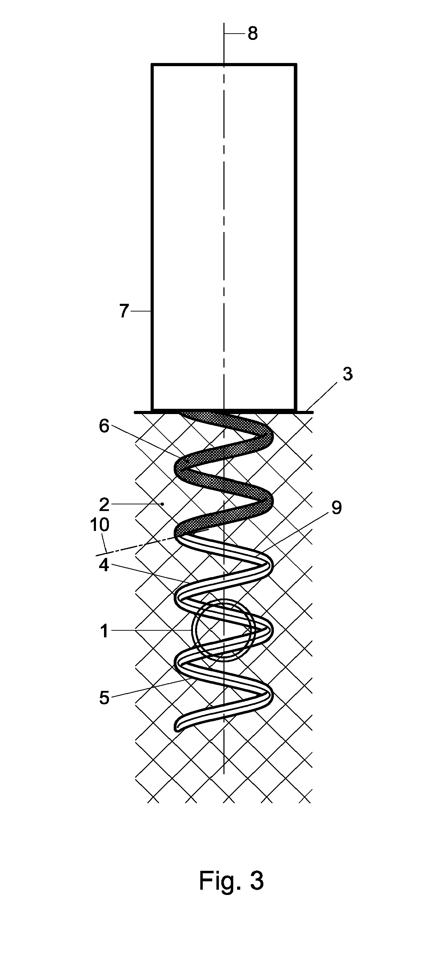

Next, the needle 4 is punctured into and through the skin tissue 3 and into underlying tissue 2 along a helical path corresponding to the helical shape of the needle 4 as is shown in Fig. 2. This is continued, preferably under ultrasound guidance, until the clamping implant has reached a position having portions (preferably at least a full winding) distally and proximally of the vein 1 to be clamped as is shown in Fig. 3, e.g. so that it encloses the vein 1 close to the junction with the deep femoral vein.

Then, the needle 4 is removed by screwing back the needle 4 along the helical path 26 through the tissue 2 underlying the skin tissue 3 (see Figs. 4 and 5). As the needle 4 is screwed back, the clamping implant 9 inside a distal end of the needle 4 is restrained against moving along with the needle 4, preferably by holding the operating member 7 engaging a proximal portion of the plunger 6 projecting from the needle 4 stationary, thereby screwing the plunger 6 further into the needle 4 as the needle 4 is screwed out. Thus, the plunger 6 urges the wire of the clamping implant 9 out of the needle 4 as the needle 4 is screwed out.

Within the framework of the invention, the plunger may also be used to advance the clamping implant out of the needle and into tissue, but preferably, the implant is not pushed into tissue while projecting from the needle. Pushing the implant out of the needle such that it pierces into the tissue would cause the leading end of the clamping implant to determine the puncturing path, so that it would not be ensured that a path would be obatained in which the clamping implant would have the desired compressing effect. Moreover, the clamping implant would most likely not reach a position bridging the vein and would cause more tissue damage.

Because the needle 4 has a helical shape, the needle 4 can be punctured to the location where the clamping implant 9 is to be delivered along a helix shaped puncture path. The coiled clamping implant 9 can be delivered from the needle 4 at the delivery location by screwing back the needle 4 and urging the clamping implant 9 out of the needle 4 by the plunger 6 as the needle 4 is screwed back. Deformation of the clamping implant 9 can remain limited to deformation to a biased state inside the lumen 5 of the needle 4 and a gradual release from the biased state inside the lumen 5 of the needle 4 to a clamping state in which the clamping implant 9 engages tissue 2 clamped thereby, as the clamping implant 9 exits the needle 4.

In unloaded condition, the plunger may have the same pitch as the needle 4 to keep friction between the plunger and the needle 4 low. The helical needle 4 is minimally invasive because the tissue damage is limited to a helical pathway 26. For minimizing tissue damage, it is advantageous that the pitch of the needle is constant over its length. The tissue 2 adjacent to the vein 1 is resilient, allowing the clamping member 9 to spring back to some extent to a less biased shape as it exits the needle 4, thereby gradually compressing the tissue 2 and pressing the vein 1 to a virtually closed condition so that at least a substantial occlusion of the vein 1 is obtained. Compression of the tissue occurs in a gradual manner and can be controlled by controlling screwing back of the needle 4.

While in the present example, a clamping implant 9 is shown which, when inserted in the needle 4 is biased such that essentially only the pitch in longitudinal direction of the central axis 8 of its helical shape is extended and reduced when the clamping implant is released from its biased shape. However, it is also possible to provide that in addition or as an alternative, the diameter of the helical axis of the clamping implant reduces when the clamping implant is released from its biased shape. The axial and radial bias may also vary over the length of the coil, for instance to provide that the clamping implant is of a spiral shape over its entire length or over a portion of its length. The clamping implant may for instance be arranged to spring or otherwise move to a configuration in which the diameter of the windings decreases towards its ends, so that the tissue volumes engaged to be clamped together are also to some extent radially compressed, which enhances axial clamping action. Further, springing back after the clamping implant has exited the needle can also be achieved in other manners than by allowing elastic deformation to spring back. The clamping implant may for instance be of a memory material that returns towards its original shape or of a material or material combination that deforms under influence of heat and/or contact with tissue liquids.

That relatively little deformation of the clamping implant is required when it is delivered is particularly advantageous for applying implants of biodegradable material. Depending on the rate of decomposition of biodegradable material, temporary closures can be applied or permanent closures can be provided without permanently leaving a clamping implant in the patient's body.

In biased condition inside the needle 4, the clamping implant 9 has a length measured along the path of its wire that is shorter than the length of the needle 4 measured along the helical axis of the needle 4. This difference in length allows the clamping implant 9 to be delivered and urged out of the needle 4 in a position in the tissue 2 in which its proximal end (the end facing the operating member 7) is spaced under the outer surface of the skin 3. The clamping implant may be a spring which, in unloaded condition has 2-4 windings and a pitch such that successive winding are in contact with each other, optionally with some pre-stress, as the windings of a double loop key ring. The implant can be loaded in the needle of a helical shape with a larger pitch under tension and will contract in the axial direction upon release.

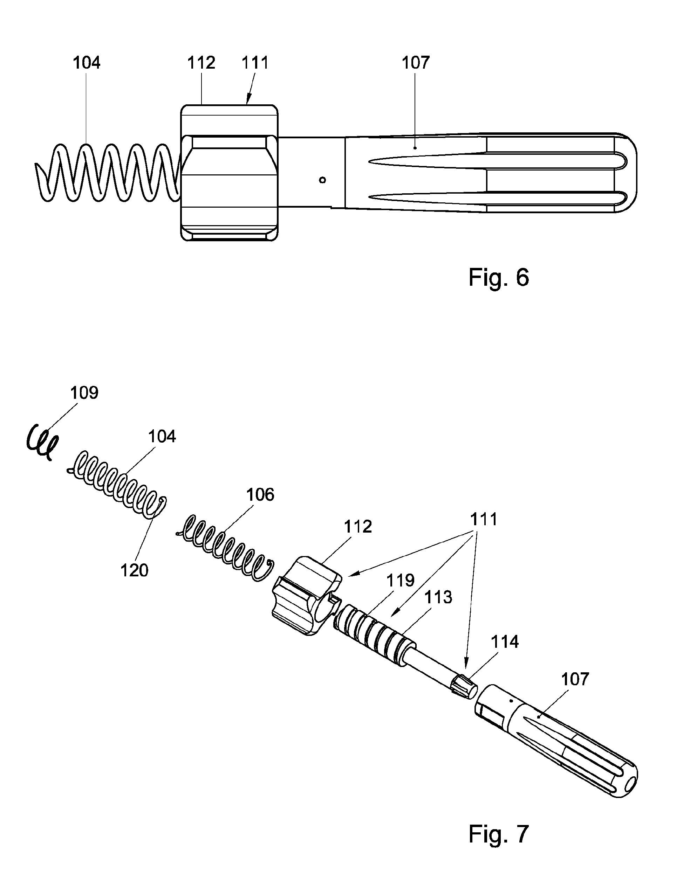

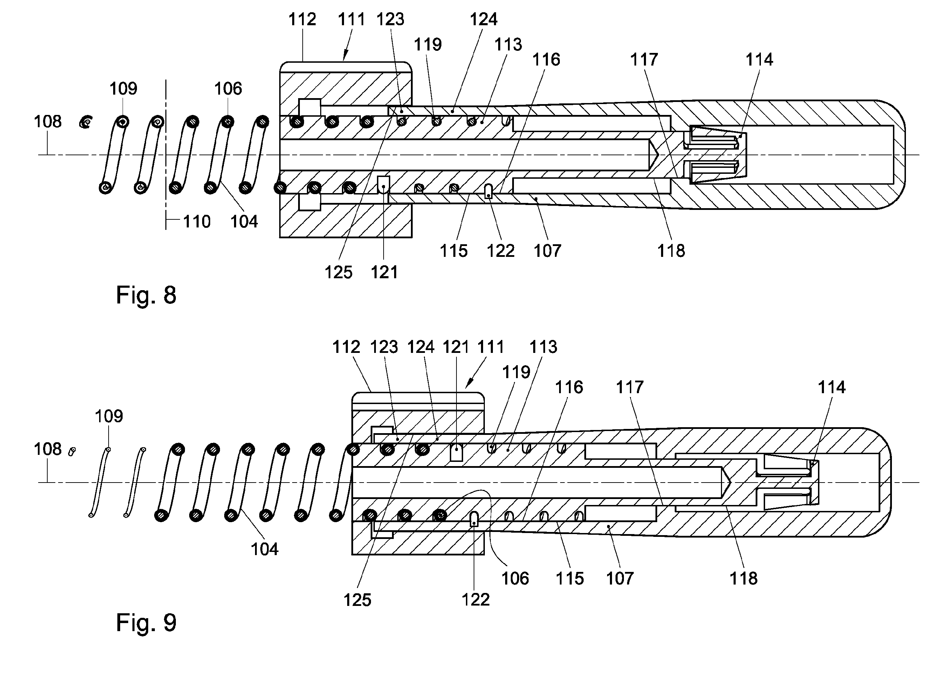

In Figs. 6-9, a second example of a tool and a system according to the invention is shown in more detail. In Figs. 8 and 9 cross-sections of the needle 104 through which the plunger 106 extends have been filled in fully in black, while in cross-sections of the needle 104 through which the clamping implant 109 extends, the cross sections of the clamping implant 109 are represented by small circles. In Fig. 7, the clamping implant 109 is shown in a condition as if inserted in the needle 104, in which the helical axis of the clamping implant 109 has a diameter and a pitch between successive windings, in longitudinal direction of its central axis, that is equal to diameter and, respectively the pitch between successive windings, in longitudinal direction of the central axis, of the helical axis of the needle 104. In unloaded condition of the clamping implant 109, its helical axis is of a shape in which at least the diameter or the pitch between successive windings, in longitudinal direction of its central axis is smaller than the diameter or, respectively the pitch between successive windings, in longitudinal direction of the central axis of the helical axis of the needle 104.

In the present example, the plunger 106 has a length such that the plunger 106 is insertable into the needle 104 up to a position in which with its distal end reaches an opening forming a distal end of the lumen in the needle 104. Thus, it can be ensured that the clamping implant can be urged out of the needle 104 and is not entrained with the needle 104 when the needle 104 is screwed out of the tissue.

However, in some cases a shorter plunger 106 may also be sufficient, for instance if it is ensured that frictional and/or interlocking engagement between tissue and the clamping implant 109 prevents the clamping member from being entrained with the needle 104 being screwed out of the tissue. Then, the plunger 106 may have a length such that the plunger 106 is at least insertable into the needle 104 beyond a proximal end of a clamping implant 109 fully inserted in a distal end portion of the needle 104, so that it is ensured that the clamping member 109 can be urged actively over at least a portion of its length. For ensuring that the distance over which the clamping implant 109 can be urged out of the needle 104 by the plunger 106 is sufficient to prevent the clamping implant 109 from being entrained when the needle 104 is screwed back, it can be provided that the plunger 106 has a length such that the plunger 106 is at least insertable into the needle 104 to a position in which its distal end is at a distance from a distal end of the needle 104 that is less than 40%, 50% or 60% of the length of the clamping implant wire measured along the path of the wire.

The tip of the needle 104 is ground to a lancet point according to industry standards.

Due to the helical shape of the needle, friction that builds up between the inner surface of the needle and the plunger is a self-reinforcing process. Overcoming friction requires that additional axial forces are exerted onto the plunger, which increases the contact pressure with which the plunger rests against the wall of the lumen inside the needle, which increase friction.

For reducing friction between the plunger and the needle, an initial fit of the plunger inside the needle with minimal stress is also advantageous. Since the force necessary to deflect the plunger over a certain angle, depends on the 4^{th} order of wire thickness, a minimal wire thickness of the plunger is helpful for keeping initial normal forces low, in particular when the shapes of the needle and of the plunger do not correspond perfectly.

In unloaded condition, the plunger 106 has helical windings having a first diameter and the helix formed by the needle 104 has a second diameter. To keep friction due to the plunger 106 leaning against a wall surface of the lumen on the outside of the helical curve in response to axial pressure exerted onto the plunger 106, the first diameter may be smaller than or equal to the second diameter. If a plunger having slightly smaller diameter than the needle is used, the plunger would fit inside the needle with additional friction between the plunger and the inside of the needle. When it is attempted to push the plunger through the needle, friction will build up and therefore the radius of the helical shape of plunger will expand. In turn, this will reduce normal forces between the plunger and the needle so that friction is reduced when the plunger is pushed into the needle.

At least the needle lumen or the plunger may be covered with a surface layer such that a frictional coefficient of the needle relative to the plunger is smaller than a frictional coefficient between the main materials of the needle and the plunger (on opposite sides of the cover or covers), e.g. smaller than metal to metal if the needle and the plunger are both made of metal.

The plunger may also be partially or completely be made of ceramic material, which is advantageous because of its low elasticity. Inadvertent deformation of the ceramic material of the plunger that would lead to deformation can be prevented by encapsulation of the plunger in the needle and in the operating member(s).

The plunger does not have to be of solid material over its entire length, but may also include a fluid column, preferably a liquid column between plugs sealed against the lumen at its proximal and distal ends. An advantage of providing that pressing force for urging the clamping member out of the needle is transferred by a fluid, at least over a portion of the plunger length, is that normal forces between the plunger and the inner surface of the lumen on the outside of the helical curve, are not increased additionally as a result of expansion of the diameter of the helical shape of the plunger.

For facilitating control over the needle 104, the tool according to the present example further comprises a needle operating member 111 engaging a proximal portion of the needle 104. In the present example, the needle operating member 111 is composed of a gripping member 112, a shank 113 and a guide member 114, which are fixedly mounted to each other, but shown separately in Fig. 7.

The needle operating member 111 is mounted to the plunger operating member 107, such that, relative to the plunger operating member 107, the needle operating member 111 is rotatable about a central longitudinal axis 108, around which the helix formed by the needle 104 extends, and axially displaceable. Thus, the needle operating member 111 and the plunger operating member 107 are guided relative to each other for following screwing motion of the plunger 106 into and out of the needle 104. For accurate guiding with little friction, the plunger operating member 107 and the needle operating member 111 have cylindrical outer and inner surface portions 115, 116 and 117, 118 with a sliding fit.

A helical channel 119 corresponding to the helix shape of the needle 104 is formed in the outer guide surface 116 of the shank 113 of the needle operating member 111. This channel 119 forms a guide for the portion of the plunger 106 that projects from the proximal end 120 of the needle 104, so that it can reliably be pushed into the needle and buckling of the plunger is avoided. The channel 119 also contains a portion of the needle 104 and keeps the needle 104 in alignment with the plunger 106. On the outside, the channel 119 is closed off by the inner guide surface 115 of the plunger operating member 107. Since neither the needle 104 nor the plunger 106 need to be shifted axially in another manner than with a screwing motion imposed by the helical shape of the needle 104, the channel for guiding the plunger may also be provided in the inner surface of the plunger operating member or in both the plunger operating member and the needle operating member.

At a needle anchoring position 121, the needle 104 is anchored to the shank 113 of the needle operating member 111. At a plunger anchoring position 122, the plunger 106 is anchored to the plunger operating member 107. If the needle operating member 111 is rotated in a left hand sense relative to the plunger operating member 107, the plunger 106 is screwed into the needle 104 is screwed into the needle 104 from a position as shown in Fig. 8 to a position as shown in Fig. 9. Although in the present example, the plunger operating member is constituted by the outer member in which the needle operating member is slidingly guided, it is also conceivable to fix the needle to the outer operating member and to fix the plunger to the inner operating member. When applied to the example shown in Figs. 6-9, this would result a swap of the needle and plunger operating functions.

To avoid premature displacement of the clamping implant 109 relative to the needle 104 during insertion of the needle 104 into the tissue towards the deployment location, the plunger 106 is releasably lockable relative to the needle 104. In the present example, this is achieved by providing the plunger operating member 107 with a locking pawl 123 at an end of a spring arm 124 that biases the locking pawl 123 into engagement with a locking recess 125. As long as the locking pawl 123 is in engagement with the locking recess 125, the needle operating member 111 is fixed relative to the plunger operating member 107. After the locking pawl 123 has been disengaged from the locking recess 125 (e.g. by pressing down the biasing arm 124 with a finger or thumb), the needle operating member 111 can be rotated relative to the plunger operating member 107, so the needle 104 can be screwed out of the tissue containing the deployment location while the plunger 106 is held essentially stationary, so that the clamping implant 109 is urged out of the needle 104.

The tool is preferably packaged with the lock in locked condition. It may be provided that the tool also locks up when the plunger is screwed into the needle over a predetermined maximum distance, for instance the distance required for completely released pushing the clamping implant out of the needle. Furthermore, it can be provided that the second lock, once locked, can not be unlocked for preventing re-use of a tool designed for use as a disposable that is not suitable for cleaning and sterilization for re-use. In the present example, this is achieved by providing that the spring arm 124 is covered by the ring 112 of the needle operating member 111 when the plunger 106 is screwed into the over the predetermined maximum distance (see Fig. 8).

For delivering a clamping implant for clamping a varicose vein, the needle may for instance have an inner diameter of at least 0.5 mm, at least 1.6 mm or at least 2 mm, and at most 2.8 mm, at most 3.2 mm or at most 5 mm. The diameter of the center line of the windings of the helix shaped needle may for instance be at least 10 mm, at least 12 mm or at least 14 mm and at most 25 mm, at most 30mm or at most 40 mm. The diameter of the center line of the windings of the clip in unloaded condition may for instance be equal to the diameter of the center line of the windings of the helix shaped needle or up to 25% or 50% smaller. The length of the helical shape in longitudinal direction of its central longitudinal axis around which the helix formed by the needle extends may for instance be at least 30 mm, at least 40 mm or at least 50 mm and at most 40 mm, at most 50 mm or at most 60 mm. The clamping implant when inserted in the lumen of the needle may for instance extend over at most 30% or 40% or 50% of the length of the needle. The clamping implement in unloaded condition may for instance extend over at least two or at least three full windings. The pitch between centerlines of successive windings of the needle (in in longitudinal direction of the central longitudinal axis of the helix), may for instance be at least 3 mm, at least 5 mm or at least 7 mm and at most 12 mm, at most 16 mm or at most 20 mm. The pitch between centerlines of successive windings of the clamping implant in unloaded condition may for instance be less than 75% or less than 50% of the pitch between successive windings of the needle. The pitch between successive windings of the clamping implant in unloaded condition may also be such that successive windings are in contact with each other, with or without a pre-stress biasing successive windings against each other.

Within the framework of the invention as set forth in the claims, many other variants of a tool and system are conceivable. For instance, instead of fitting in the needle completely, the implant can fit inside the needle only partially, for instance because a distal tip forms a piercing point for piercing the helical trajectory through the skin and underlying tissue. The clamping insert can be arranged to contract on release, so that also an axial contraction of engaged tissue is achieved. The clamping implant can be applied to humans or animals. Instead of a single integral piece, the clamping implant can also consist of more than one part which may be temporarily or permamently connected to each other.

## Claims

1. A tool for delivering and applying a clamping implant (9; 109) via a puncture in skin tissue (3) and underlying tissue (2) of a patient, comprising:
a hollow needle (4; 104) bounding a lumen (5; 105) and forming a helix,
a plunger (6; 106) of which a distal portion extends in longitudinal direction inside the lumen (5; 105), and
a plunger operating member (7; 107) engaging a proximal portion of the plunger (6; 106) projecting from the needle (4; 104), for screwing the plunger (6; 106) further into or out of the needle (4; 104) by screwing motion of the needle (4; 104) relative to the plunger (6; 106).

2. A tool according to claim 1, wherein the plunger (6; 106) has a length such that the plunger (6; 106) is at least insertable into the needle (4; 104) with its distal end up to an opening forming a distal end of the lumen (5; 105) in the needle (4; 104).

3. A tool according to claim 1 or 2, further comprising a needle operating member (111) engaging a proximal portion of the needle (104).

4. A tool according to claim 3, wherein the needle operating member (111) is mounted to the plunger operating member (107), such that, relative to the plunger operating member (107), the needle operating member (111) is rotatable about a central longitudinal axis (108) around which the helix formed by the needle (104) extends and is axially displaceable.

5. A tool according to claim 4, wherein the plunger operating member (107) and the needle operating member (111) have cylindrical outer and inner surface portions (115, 116; 117, 118) with a sliding fit.

6. A tool according to claim 5, having a helical channel (119) corresponding to the helix, wherein said helical channel (119) is formed in at least said inner or outer surface (115, 116), at least a portion of the channel (119) forms a continuation in line with the lumen (105) in the needle (104) and wherein the plunger (106) extends partially into the needle (104) and partially within said channel (119).

7. A tool according to any of the preceding claims, wherein the plunger (106) is releasably lockable relative to the needle (104).

8. A tool according to claim 7, wherein the plunger (106) is releasably lockable relative to the needle (104) in a load position leaving a distal end portion of the needle (104) lumen (105) free for accommodating a clamping implant (109).

9. A tool according to claim 8, wherein the plunger (106) is non-releasably lockable relative to the needle (104) in a position screwed into the needle (104) from said load position for urging a clamping implant (109) out of the needle (104).

10. A tool according to any of the preceding claims, wherein in unloaded condition, the plunger (6; 106) has helical windings having a first diameter and wherein the helix formed by the needle (4; 104) has a second diameter, the first diameter being smaller than or equal to the second diameter.

11. A tool according to any of the preceding claims, wherein the needle (4; 104) and the plunger (6; 106) are covered with a surface layer such that a frictional coefficient of the needle (4; 104) relative to the plunger (6; 106) is smaller than a frictional coefficient between the main materials of the needle (4; 104) and the plunger (6; 106).

12. A tool according to any of the preceding claims, wherein the plunger includes a fluid column, preferably a liquid column between plugs sealing off the lumen at its proximal and distal ends.

13. A system comprising a tool according to any of the preceding claims, further comprising a coiled wire clamping implant (9; 109) inserted or insertable into the lumen (5; 105) of the needle (4; 104), wherein the helix of the needle (4; 104) has a pitch and a diameter, wherein the coiled wire of the clamping implant (9; 109) in unloaded condition has at least a pitch smaller than the pitch of the helix or a diameter smaller than the diameter of the helix, and wherein the coiled wire of the clamping implant (109) has a length measured along the path of the wire that is shorter than the length of the needle (104) measured along the helix of the needle (104).

14. A system according to claim 13, wherein the plunger (6; 106) has a length such that the plunger (6; 106) is at least insertable into the needle (4; 104) beyond a proximal end of a clamping implant (9; 109) fully inserted in a distal end portion of the needle (4; 104).

15. A system according to claim 14, wherein the plunger (6; 106) has a length such that the plunger (6; 106) is at least insertable into the needle (4; 104) to a position in which its distal end is at a distance from a distal end of the needle (4; 104) that is less than 60% of the length of the clamping implant (9; 109) wire measured along the path of the wire.

16. A method for delivering and applying a coiled wire clamping implant (9; 109) via a puncture in skin tissue (3) and underlying tissue (2) of a patient, comprising puncturing a hollow needle (4; 104) bounding a lumen (5; 105) and having a longitudinal axis forming a helix into the skin tissue (3) and the underlying tissue (2) along a helical path (26) corresponding to the helix of the needle (4; 104), removing the needle (4; 104) by screwing back the needle (4; 104) along the helical path (26) through the underlying tissue (2) and the skin tissue (3), and, as the needle (4; 104) is screwed back, restraining the clamping implant (9; 109) inside a distal end of the needle (4; 104) against moving along with the needle (4; 104) by restraining a plunger (6; 106) of which a distal portion extends in longitudinal direction inside the lumen (5; 105) and against following the needle (4; 104), thereby screwing the plunger (6; 106) further into the needle (4; 104), such that the plunger (6; 106) urges the wire of the clamping implant (9; 109) out of the needle (4; 104) as the needle (4; 104) is screwed back.
